# EUROPEAN PATENT APPLICATION

(11) **EP 1 433 418 A1**
(43) Date of publication of application: **30.06.2004**
(21) Application number: 03257510.2
(22) Date of filing: 28.11.2003
(51) Int. Cl.: A61B 5/103

(54) **Skin diagnostic imaging method and apparatus**

(30) Priority: 27.12.2002 US 436902 P
(71) Applicant: UNILEVER PLC, London EC4P 4BQ (GB); UNILEVER N.V., 3013 AL Rotterdam (NL)
(72) Inventor: Eddowes, Miles Hugh Unilever R&D, Wirral, Merseyside, CH63 3JW (GB); Kennedy, Chrisine Elizabeth, Trumbull, Connecticut 06611 (US); Cahill, John Gilbert, Edgewater, New Jersey 07020 (US); Shirsat, Sanjay A, Edgewater, New Jersey 07020 (US)
(74) Representative: Mulder, Cornelis Willem Reinier, Dr.

(57) **Abstract**

A method and apparatus is provided for identifying imperfections in a person's facial, forearm or hand skin and for recommending an appropriate remedial cosmetic. The method includes providing an apparatus having a programmable computer, a camera connected to the computer, at least one visible wavelength light source for separately generating at least two different color images and at least one ultraviolet wavelength light source for an ultraviolet light image. Further, the method includes placing the ultraviolet and color images into a program of the computer and processing the images for pinpointing areas of skin requiring preventative treatment including those with skin damage. A remedial set of cosmetic products can thereby be recommended.

## Description

The invention concerns a method and apparatus for identifying imperfections in a person's skin, and for recommending the appropriate remedial cosmetics.

Cosmetic shelves are filled with a plethora of skin treatment products. Consumers suffer from in-store confusion in selecting the product most effective for their face and hands.

Systems have been developed to educate consumers in selecting their optimum product. For instance, U.S. Patent 5,622,692 (Rigg et al.) describes an in-store system developed by the Elizabeth Arden Company called Custom Color@ for identifying a customer's perfect matching facial foundation. A spectrophotometer first reads the natural color of the skin. Information from this reading is transmitted to a computer which operates a machine dispensing a liquid foundation matching the measured color.

Other than foundations, women require assistance in selecting treatment products. Before an appropriate product can be identified, it is necessary to first understand the skin condition of a particular customer. Cosmetics producers have experimented with in-store systems for providing such information.

Many are simply based upon questions presented to the customer. Clinique has operated for many years with an in-store display. Questions are asked regarding a customer's skin oiliness, moisture and preferences. Procter & Gamble has launched the Olay® Beauty Imaging System (BIS) in stores located in the U.K., U.S. and Japan. BIS incorporates a library of several thousand facial images which have been analyzed, and statistical models developed for age-dependent changes in skin features for a variety of racial groups.

In a store setting, customers are photographed under visible light. Based upon the developed statistical models, the customer's skin is scored and placed on a graph showing how the score relates to a predicted level of skin condition. Products can then be recommended. BIS is not consumer friendly, and requires expensive equipment. Features of the system are disclosed in WO 01/91600 and an article by Miyamoto and Hillebrand titled "The Beauty Imaging System: For the Objective Evaluation of Skin Condition" appearing in the Journal of Cosmetic Science, pages 62-65, January-February Edition 2001.

Ultraviolet light imaging to reveal sub-surface sun damage has been detailed by J. Niamtu in an article titled "Digitally processed ultraviolet images: A convenient, affordable, reproducible means of illustrating Ultraviolet clinical examination" appearing in Dermatologic Surgery, Vol. 27 (Issue 12), page 1039, December 2001 published by Blackwell.

Canfield Scientific, Inc. sells a kit for photographing faces which includes a camera with a pair of lights changeable from visible to UV, and a program which coordinates the flashing of lights with camera action.

Despite the aforementioned advances in the art, there remains a need for an in-store system sufficiently simple for a customer to operate without requiring store personnel help. Moreover, the system needs to provide the customer with pictorial feedback identifying the problem areas and the degree of age and photo-damage, all of which provides an objective assessment of skin condition in a rapid manner.

In a first aspect, a method is provided for identifying imperfections in a person's skin and for recommending appropriate remedial cosmetics, the method including:
(i) providing an apparatus which includes:
   (a) a program for storing information;
   (b) a camera connected to the program;
   (c) at least one visible wavelength light source associated with the camera for separately generating at least two different color images; and
   (d) at least one ultraviolet wavelength light source associated with the camera for generating an ultraviolet light image;
(ii) positioning a body part of a person (such as the face, forearm or hand) within a photographical area of the light sources and camera;
(iii) obtaining the at least two different color and ultraviolet light images of the body part which information is placed into the program;
(iv) processing the images via the program and thereby identifying personal aspects selected from the group consisting of skin attributes, signs of skin damage and defects; and
(v) recommending a cosmetic product most suitable to the person based upon the identified personal aspects.

In another aspect of the invention, there is provided an apparatus for identifying imperfections in a person's skin and for recommending appropriate remedial cosmetics, wherein the apparatus includes:
(a) a program for storing information;
(b) a camera connected to the program;
(c) at least one visible wavelength light source associated with the camera for separately generating at least two different color images; and
(d) at least one ultraviolet wavelength light source associated with the camera for generating an ultraviolet light image.

Further features and benefits of the present invention will become more readily apparent through consideration of the drawings, in which:
- Fig. 1 is a front perspective view of an equipment set according to one embodiment of the present invention;
- Fig. 2 is an elevational view of a booth housing a similar equipment set embodiment as in Fig. 1;
- Fig. 3 is a half-face analysis image printout according to one embodiment of the invention; and
- Fig. 4 is a full-face analysis image printout according to a second embodiment of the invention.

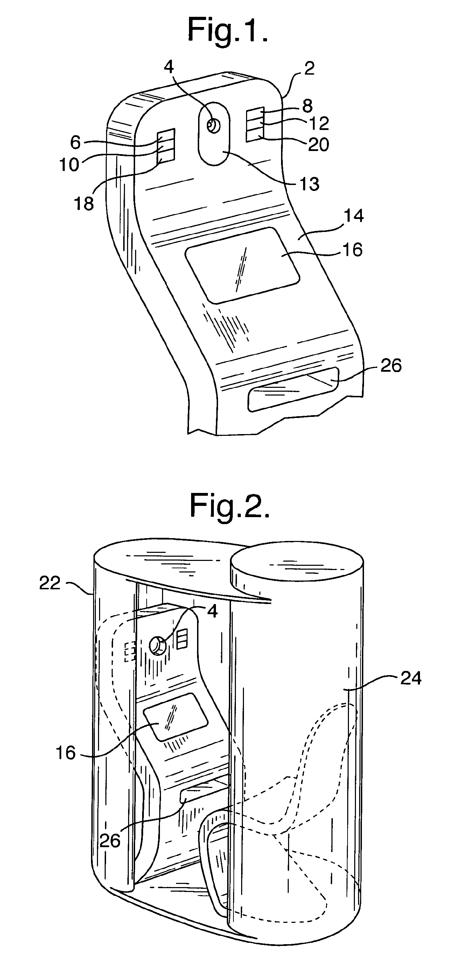

According to an aspect of the present invention, there is provided an apparatus shown in Fig. 1 to practice the method for identifying skin imperfections. Console 2 frames the equipment. Within the console is positioned a camera 4.

Many types of cameras are suitable for this invention. A useful example is the Fuji S2 digital camera. This employs a super CCD sensor, and delivers a high resolution of 6.1 million pixels (3040 X 2016). The S2 camera is based upon a Nikon N60 body, and can accept essentially all Nikon® F-mount lenses. A FireWire (IEEE 1394) Interface can be utilized to download the images from the camera to a hard drive of a computer. A suitable lens is an AF Micro-Nikkor 60 MM 1:2.8 D.

Alternative to the CCD detection system is a CMOS imaging array based system which may be utilized for low cost arrangements.

A pair of ultraviolet wavelength generating lights 6, 6' and a set of visible wavelength generating lights 10, 11, 12 and 10', 11' and 12' fitted with red, green and blue filters are arrayed one of each kind flanking left and right sides of the camera. A computer 14 processes information received from the camera. A face of the customer based upon the camera information is displayed on a monitor screen 16. A mirror 13 draws the customer's attention to face the camera.

Although a monitor screen for displaying the person's face or hands is useful and interacts efficiently with the program, in some embodiments this feature may not be necessary.

The lights can be of the standard flash photography variety. Fluorescent tubes, halogen and tungsten filament bulbs may be utilized.

A suitable flash photography light is the NOMFH 2 - Novaflex® Auto Duo Flash Twin Head Macro Flash connected to an AFNME - Novaflex® Adaptor for Nikon® Auto Focus Camera generator. Alternatively the flash unit can be a Nikon® Speed Light SB 80 DX. The generator may be held together with a Nikon® AS-10 TTL Multi Flash Adaptor and the flash units connected with a Nikon® SC 18 Multi Flash Cord to ensure a completed circuit.

The ultraviolet wavelength generating source ordinarily is one of the aforementioned visible light flash units which have been adapted with a UV filter. For purposes of this invention ultraviolet wavelength is defined as ranging from 200 to 400 nm, preferably from 200 to 380 nm. Visible wavelength light is defined as ranging from 410 to 700 nm.

Different color images utilized for this invention are best provided by separate visible white light lamps covered by filters to generate each of red, green and blue light. Use of a sequence of individual colored separate light images is particularly of importance when low performance (low cost) visible color or monochromatic (black & white) cameras are employed in the apparatus. With these cameras, successive image frames of a static object are taken. Each color frame in turn can be combined into a single color image with potential for a very high level of color discrimination. Excellent combination images are thereby achieved for viewing. For analysis purposes, it may be sufficient to utilize only separate red and blue or separate red and green light generated images.

An alternative approach to generating separte red, green and/or blue images is to employ a white light for illumination, and sequentially placing different color band pass filters in front of the camera.

A different way of increasing the ability of a low cost color imaging system to discriminate a color difference is to include a calibrated color chart in part of the image frame of the body part. The chart can be used for post-acquisition of the image to correct for deficiencies or inconsistencies. These problems may arise from the spectrum of the lighting or the color calibration of the camera. Charts are available from Gretag/Macbeth of New Windsor, NY, under the Color Checker@ trademark.

Additional lights can be employed which generate near infrared wavelengths (greater than 700 to 1000 nm). Near infrared light is useful to correct and enhance the visible image. Natural skin has only very weak chromophores in the near infra-red part of the spectrum. The resulting photos contain predominantly backscattered light information relating almost exclusively to skin topography and geometry of the illumination. This light can therefore be used to correct for shadows and other limitations in the visible or UV lighting.

Furthermore, differences between the visible photos and based on those photos, expectations of UV or near infrared wavelengths are useful to determine cosmetics (e.g. foundations, lipsticks) worn by a customer. Correction can then be programmed to disregard pre-applied cosmetic make-up. This ensures accurate product recommendations. Fig. 1 illustrates the near infrared wavelength light 18, 18' as an additional lamp with appropriate filter flanking the camera.

Manufacturers such as Roithner Lasertechnik based in Vienna, Austria, supply high power Light Emitting Diodes suitable for generating all the ultraviolet, visible and near infrared wavelengths required within this invention.

Polarizing filters can be placed over any of the light sources and the camera. By the utilization of copolarized lighting conditions, skin attributes such as "ashiness" and "flakiness" can be highlighted for computerized measurement. Cross polarized lighting can enhance chromophores apparent in deeper skin layers such as the basal of the epidermis or of the dermis itself.

Full front face image is the most preferred view. However, side (profile) views ranging in angle from about 30 to about 120°, particularly a 45° angle may also be useful. Indeed, a combination of full front face and side face view can be analyzed and also presented as a printout to the customer. Additionally, an exploded view of a particular area of the face or hands can be taken. The crows foot area along the eyes is a top candidate for an exploded view.

Particularly advantageous is a combination of three cameras focused on a customer to obtain full front, side and exploded views. Alternatively, the same image set can be taken using a moveable single camera. Analysis of the crows foot and other areas requiring enhancement of topographical features can be accomplished by camera positioning at different angles catching different light reflections. A Canny filter is an image analysis method that can extract edge-like features within an image.

An image analysis routine comprising a Canny filter may be used on two images of the same skin area, but with illumination at two different angles. For instance, suitable are the zero-degree "perpendicular" angle and the 45 degree angle. Ratios of the Canny algorithm computed wrinkle length taken under these two different lighting conditions is a measure of how visible a wrinkle appears.

The method of the present invention requires that a customer's face, forearm or hands be positioned in a photographical area of the camera and lights. Proper positioning can be accomplished in a variety of ways.

One method of proper positioning is the use of a chin rest. A customer places their chin upon the chin rest. A pressure sensor under the chin rest can activate the system to initiate flashing of the proper lights in an appropriate sequence and for the camera to record the customer's appropriate body parts. Another embodiment may involve positioning by means of a chair oriented in an appropriate direction. A pressure sensor on the chair may activate the system. Motion sensors may also be utilized for initiating the lights/camera sequence.

Sensors can initiate stored verbal directions when a customer comes within range of the system.

Fig. 2 illustrates a booth 22 for housing the console 2 in a private setting. In this embodiment, a chair 24 serves as the positioning device. A motion detector within the console identifies the presence of a customer. An audio system is triggered by the motion sensor requesting the customer to touch the monitor screen, thereby beginning the diagnostic procedure.

Alignment of the camera with the customer's face is desirable for an accurate and repeatable assessment of facial skin using digital imaging. A variety of mechanisms can achieve this result. For instance, fiber optic focusing devices can be utilized that project a grid on an object such as the face being imaged. There also is the possibility to project an image grid onto a customer's face while looking into a mirror or a camera, with a live video feed relayed to the customer in a virtual mirror mode. This allows the customer to align her face properly with the projected image for subsequently obtaining a diagnostic true image of the face.

Another alignment option is to use a digital video that can capture a particular length of image footage. This is combined with an algorithm that can be used to analyze the captured frames to pick one that is aligned correctly. Still a further possibility is the use of a motion detection sensor for matching camera to the customer's face.

Yet another possibility is for the camera to be automatically or manually oriented such that face or other body part is placed within the image frame without the need for the customer to take great care in positioning themselves. Auto-locating may be by imaging of an area much larger than the face or body part itself. It may involve computer algorithms to detect features on the body part which define exactly where the regions of interest should lie within the image frame.

Advantageously a customer is commanded (either through written or sound delivered) instructions to close their eyes. By requiring closed eyes, there is avoided any jolt movement on face or body when the lights flash for the photographic moment. Consequently the customer is posed with a more standard visage than when the camera records after an open eyed variable response from different customers as the lights flash on.

An alternative to customer instruction for closing of eyes before the photographic moment is to position a customer's face in alignment with a baffle or shield along the eye areas. In this arrangement, the eyes will not be exposed to any light flash which might precipitate a jolt movement.

Ordinarily the method of the present invention will involve three flashes. One of these will be an ultraviolet wavelength flash, and the other a set of the colored visible wavelength flashes of light. Ultraviolet and color composite visible images are then displayed to the customer on the monitor screen 16. The ultraviolet photo image highlights damage caused by sun exposure and is not readily seen in a visible photograph. On the other hand, the visible photographs processed through the program of the present invention highlight fine lines and wrinkles.

Computerization analysis made from the ultraviolet image can determine the presence of sunscreen (e.g. cosmetics) on the customer's face, and can compensate the visible images for the cosmetic layer.

Near infrared images can also be taken to compensate for shadows and limitations, with the lighting as well as identify the presence of cosmetics on the customer's face. The ultraviolet lights are particularly useful around the lips and forehead to reveal wrinkles.

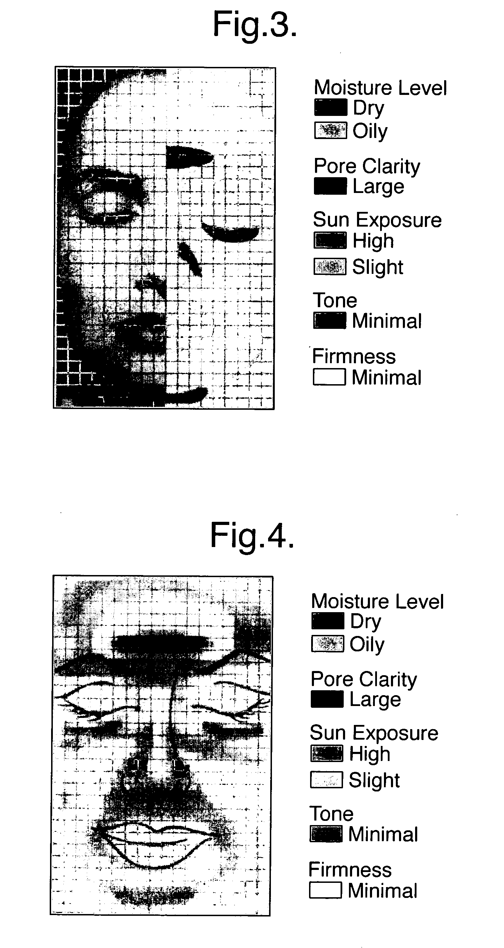

The present system is capable of providing a printout score covering skin attributes such as the five signs of healthy skin, any damage or defects. The five attributes include moisture level, pore clarity, sun exposure, tone and firmness. Fig. 3 is a half natural and half schematic image of a customer. Image and other diagnostic features of the present invention identify on the half schematic those areas of a customer's face where remedial cosmetic treatment is needed with respect to each of the five signs of healthy skin. By the term "schematic" is meant a pictorial representation showing areas of skin requiring treatment.

The image of Fig. 3 is calculated by the program and delivered to the monitor screen. Customers may have a printout of their ultraviolet, visible, schematic image and computer generated personalized cosmetic treatment regime through a color printer with outlet 26.

Fig. 4 illustrates a second embodiment wherein the customer's face is in total schematic form. Similar to the half schematic form, the image of Fig. 4 highlights problem areas of the customer's face requiring remedial cosmetic attention. The computerized recommendations let the customer know how their skin compares on a normative scale relative to their age, ethnic background and other preprogrammed baseline information. Measured parameters can be stored within the local computer or a distant computer network. Customers can be provided at a future time with the parameters for purposes of showing progression or remediation of the skin's conditions.

Additional sensors or probes can be used in conjunction with, or incorporated into, this system for spot measurements on the skin. These include electrical conductance probes for moisture readings, ballistometers for mechanical properties and skin translucency sensors or colorimeters for further measures of the skin's color. Spot measurements can be used to augment information derived from the images and questionnaires or, in the case of visible colors, can be used to calibrate a single point on the image. Other characteristics within the image, such as points of color, can then be referred to as differences relative to the calibrated reference point. Particularly in the case of low cost systems, this will make for a more accurate reading than can be inferred straight from pixel values generated by a camera.

In one embodiment, the system and program recommendation need not rely solely upon camera image information. A customer can be requested to provide input on a series of questions which will help diagnose the skin and also to suggest the most appropriate cosmetic product to achieve the signs of healthy skin.

The method of the present invention and associated apparatus may further include the delivery of a discount coupon for purchase of products recommended as a result of the image analysis. One method of instantly delivering a coupon is through printout from the printer that may be associated with the apparatus.

The term "comprising" is meant not to be limiting to any subsequently stated elements but rather to encompass non-specified elements of major or minor functional importance. In other words the listed steps, elements or options need not be exhaustive. Whenever the words "including" or "having" are used, these terms are meant to be equivalent to "comprising" as defined above.

## Claims

1. A method for identifying imperfections in a person's skin and for recommending appropriate remedial cosmetics, the method comprising:
(i) providing an apparatus which includes:
(a) a program for storing information;
(b) a camera connected to the program;
(c) at least one visible wavelength light source associated with the camera for separately generating at least two different color images; and
(d) at least one ultraviolet wavelength light source associated with the camera for generating an ultraviolet light image;
(ii) positioning a body part of a person within a photographical area of the light sources and camera;
(iii) obtaining the at least two different color and ultraviolet light images of the body part which information is placed into the program;
(iv) processing the images via the program and thereby identifying personal aspects selected from the group consisting of skin attributes, signs of skin damage and defects; and
(v) recommending a cosmetic productmost suitable to the person based upon the identified personal aspects.

2. The method according to claim 1 further comprising the step of shielding the person's eyes from flashes of light emanating from the light sources.

3. The method according to claim2 wherein the step of shielding comprises instructing the person to close their eyes at a point prior to obtaining of an image of the person's face.

4. The method according to claim 2 wherein the step of shielding is achieved by a baffle placed in a path between the person's eyes and a flashing light source of the apparatus.

5. The method according to any of thepreceding claims wherein the at least two different color images are generated separately from a red wavelength source, a green wavelength source and a blue wavelength source.

6. The method according to any of the preceding claims wherein the at least two different color images are generated by placing different color band pass filters in front of the camera and flashing a white light source.

7. The method according to any of the preceding claims wherein the apparatus further comprises at least one near infrared wavelength light source and the step of generating a near infrared wavelength light image of the body part which information is placed into the program.

8. The method according to any of the preceding claims wherein the apparatus further comprises a monitor screen for displaying the person's body part, the monitor screen being connected to the program.

9. The method according to any of the preceding claims wherein the apparatus further comprises a printer, and the method further comprises delivering through the printer a discount coupon for purchase of the recommended product.

10. A method according to any of the preceding claims further comprising printing an image of the person's body part in schematic mode with areas highlighted to identify need for remedial cosmetic product treatment.

11. An apparatus comprising:
(a) a program for storing information;
(b) a camera connected to the program;
(c) at least one visible wavelength light source associated with the camera for separately generating at least two different color images; and
(d) at least one ultraviolet wavelength light source associated with the camera for generating an ultraviolet light image.

12. The apparatus according to claim 11 further comprising a printer, the program being programmed to print via the printer a discount coupon for purchase of a recommended product.

13. The apparatus according to claim 11 or claim 12 further comprising a monitor screen for displaying a body part of a person, the monitor screen being connected to the program.

14. The apparatus according to any of claims 11 to 13 wherein the at least two color images are generated separately from a red wavelength source, a green wavelength source and a blue wavelength source.

15. The apparatus according to any of claims 11 to 14 further comprising at least one near infrared wavelength light source.
